# EUROPEAN PATENT SPECIFICATION

(11) **EP 3 585 315 B1**
(45) Date of publication and mention of the grant of the patent: **30.08.2023**
(21) Application number: 18712458.1
(22) Date of filing: 22.02.2018
(51) Int. Cl.: A61F 2/28, A61F 2/30

(54) **SYSTEM FOR MAKING A CRANIAL OPENING IN A LIVING BEING**
SYSTEM ZUR HERSTELLUNG EINER SCHÄDELÖFFNUNG IN EINEM LEBEWESEN
SYSTÈME DE RÉALISATION D'UNE OUVERTURE CRÂNIENNE DANS UN ÊTRE VIVANT

(30) Priority: 23.02.2017 IT 201700020563
(43) Date of publication of application: 01.01.2020
(73) Proprietor: Intelligenza Trasparente S.r.l., 20900 Monza (Monza E Brianza) (IT)
(72) Inventor: DI MECO, Francesco, 20900 Monza (Monza E Brianza) (IT); PRADA, Francesco Ugo, 20900 Monza (Monza E Brianza) (IT); SOLBIATI, Marco, 20900 Monza (Monza E Brianza) (IT); ROTILIO, Alessandro, 20900 Monza (Monza E Brianza) (IT)
(74) Representative: Tansini, Elio Fabrizio
(86) International application number: PCT/IB2018/051102
(87) International publication number: WO 2018/154477

(56) References cited:
- DE-A1- 10 006 851
- DE-A1-102015 205 538
- GB-A- 2 379 185
- US-A1- 2017 024 501

## Description

### FIELD OF APPLICATION

The present invention relates to a system, as defined in the appended claims, for producing a cranial operculum for a living being.

In particular, the present invention relates to a system for producing a cranial operculum for a living being during a surgical procedure involving a craniotomy.

### PRIOR ART

Most neurosurgical procedures at the cranial level entail removing a bone flap of varying dimensions, adapted to perform the procedure, which is set back in place at the end of the procedure. It is often necessary to carry out surgical procedures intended to reconstruct the cranial wall by means of an artificial plate or operculum to fill in a bone gap in the skull (cranioplasty). For example, various types of surgical procedures on the human brain require the removal of a portion of the skull. By way of non-limiting example, surgical procedures to remove brain tumours, surgical procedures to reduce brain swelling (e.g. oedemas, neoplasms, infections, vascular lesions), and surgical procedures necessary to reconstruct damaged parts of the skull.

In surgery involving first removal and then reconstruction with cranioplasty prepared in the pre-surgery phase, precision is a must. If, due to a technical error, the craniectomy is larger than the artificial cranial operculum, it can prove very difficult to remedy this and the final result can be highly disappointing.

Before entering the operating room, neurosurgeons are in the habit of examining all or part of the neuroradiological material produced. Recently, this has been greatly helped by routine use of a neuronavigator, whose multiplanarity and the possibility of creating visualisation planes at varying depths from the surface makes it possible to devise mental simulations of surgical planning.

Thanks also to the possibility of overlaying computed tomography (CT) on brain magnetic resonance (MR) images in a single examination, the neuronavigator makes it possible to easily understand the best way to carry out a surgical approach.

The use of a neuronavigator is to be considered essential in all cases of removal and reconstruction performed during a single surgical procedure.

At present, the use of diagnostic imaging methods, i.e. techniques performed in order to obtain images that provide diagnostic information, plays an essential role in the performance of neurosurgical procedures, making it possible to plan a procedure better and enabling an anatomic and functional definition of the damaged region of the brain. Furthermore, diagnostic imaging methods can help to guide the neurosurgeon during the procedure. For example, intra-surgery uses of ultrasound in neurosurgery, in which the ultrasound probe is placed directly on the brain, enables an excellent definition of the anatomical situation in the surgical field.

The use of diagnostic imaging methods also continues in the post-surgery phase, in order to assess the entity of the brain resection and the effectiveness of adjuvant therapies. Such therapies, however, may have side effects, and are time-consuming and expensive in terms of financial and human resources. Furthermore, some patients may not respond to a certain procedure and/or adjuvant therapies, resulting in the need to continue monitoring and identifying the treatment area. Early identification of these cases, in addition to improving the treatment of those patients, would result in considerable cost savings.

Although ultrasound scanning is a tool widely used in the field of general radiodiagnostics, is limited to just a few areas in brain diagnostics. In fact, in post-surgery follow-up examinations, the highly hyperechogenic nature of the skull (i.e. it generates an echo when struck by an ultrasound beam during an ultrasound examination) prevents the ultrasound from penetrating into the cranial cavity, with the exception of the ocular region and the temporal acoustic window.

Furthermore, there are technologies which enable the irradiation of tissues of living beings with high-intensity focused ultrasound beams for the treatment of functional pathologies or in order to treat brain lesions. Focused ultrasound beams have the further effect on the brain of opening the blood-brain barrier and allowing better drug absorption.

The repositioning of the cranial bone flap removed following the neurosurgical procedure in fact creates an obstacle to ultrasound and precludes follow-up examinations of the patient using ultrasound or treatments with focused ultrasound beams.

Furthermore, though occasionally available, the present ultrasound treatment methods capable of passing through the skull do not yet enable an effective treatment and entail a very high delivery of energy.

GB2379185 discloses a system for producing a cranial operculum based on the shape of a cranium already comprising a missing portion.

One object of the present invention is to provide a system for producing a cranial operculum for a living being that makes it possible to drastically reduce fabrication times and improve precision in the production thereof.

Another object of the present invention is to provide a system for producing a cranial operculum for a living being which enables it to be produced at the time of surgery.

Another object of the present invention is to provide a system for producing a cranial operculum for a living being that enables it to be produced based on planning carried out on a planning module that uses a CT or RM image as input.

A further object of the present invention is to enable an artificial cranial operculum to be made with rigid, biocompatible, sterilisable, ultrasound-transparent materials.

Another object of the present invention is to enable an effective use of ultrasound in the field of post-surgery brain diagnostics.

A further object of the present invention is to enable the claimed device to be used as an interface on which other diagnostic and/or therapeutic devices can be easily applied, so as to enable follow-up examinations to be performed in the post-surgery period in order to monitor the course of the disease or recovery from the brain lesion and also for the administration of therapies.

Another object of the present invention is to provide a system for producing a cranial operculum for a living being that enables specific surgical strategies to be devised rapidly and cost-effectively by reproducing the area of intervention concerned and simulating thereupon the procedure in the pre-surgery phase.

A further object of the present invention is provide a rapid, easy-to-implement system for simulating the production a cranial operculum.

### SUMMARY OF THE INVENTION

These and other objects are achieved by a system for producing a cranial operculum for a living being according to what is disclosed in claim 1.

Advantageous aspects are disclosed in dependent claims 2 to 10.

The invention achieves a plurality of technical effects:
- efficient, rapid production of an artificial cranial operculum to replace a bone operculum removed from a skull during a neurosurgical procedure which comprises a craniotomy;
- in particular, it achieves the production of the cranial operculum during the neurosurgical procedure;
- high flexibility in the production of the operculum, both in the choice of the materials used and in the possibility of including various diagnostic instruments capable of monitoring the vital characteristics of the brain in the post-surgery phase;
- possibility of producing an artificial operculum using ultrasound-transparent materials, thus facilitating the follow-up phase;
- drastic reduction in surgery times (and, consequently, in the likelihood of infections due to prolonged exposure), since the procedure is limited to the implantation of a prosthesis made to measure during the procedure itself;
- from the viewpoint of mechanical performance, a better performing skull prosthesis, devoid of asymmetries in thickness or deformations, and without the presence of air bubbles, thus eliminating the risk of inflammatory reactions due to bacterial colonisations, and highly ultrasound-compatible;
- possibility of carrying out pre-surgery planning, in particular, based on diagnostic images of the living being;
- simulation of the production of a cranial operculum that is precise in terms of structure and size.

The simulation step performed by the invention carries out technical functions typical of modern engineering work. It provides, in the pre-surgery phase, realistic predictions of the cranial zone involved in the procedure and of the optimal shape and size of the cranial operculum that must be accommodated, and thereby ideally enable the operculum to be developed so accurately that the possibilities of success of a prototype can be assured prior to its actual production.

The technical effect of this result grows with the speed of the simulation, since this enables a broad range of designs to be tested and examined virtually in order to evaluate the correspondence before the process of fabrication of the cranial operculum begins.

Without technical support, an advanced test on a complex operculum and/or an accurate selection among many different designs would not be possible, or at least not possible within a reasonable time.

The technical effects/advantages cited and other technical effects/advantages of the invention will emerge in further detail from the description provided herein below of an example embodiment and they are provided by way of approximate and non-limiting example with reference to the attached drawings.

### BRIEF DESCRIPTION OF THE DRAWINGS

Figure 1 shows a functional block diagram of the system for producing an artificial cranial operculum for a living being according to the invention.
Figure 2 shows a block diagram of the processing unit of the system of figure 1.
Figure 3 schematically shows a lateral view of a patient following the removal of a cranial operculum from the skull;
Figure 4 schematically shows a lateral cross section of an artificial cranial operculum.
Figure 5 schematically shows an orthogonal view of an artificial operculum with multiple fixation fins and with a single fixation fin.

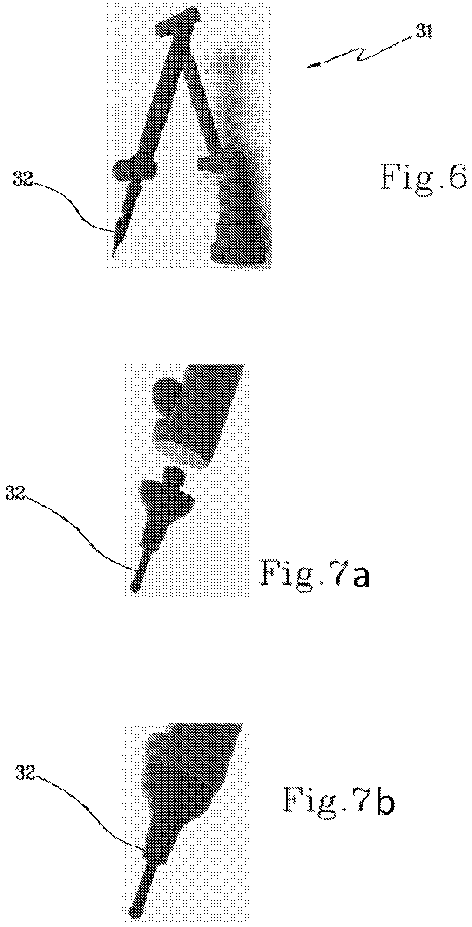

Figure 6 shows a three-dimensional detection arm with an attached probing tip.
Figures 7a and 7b show the probing tip of figure 6 respectively in an exploded view and attached to the detection arm.
Figure 8 shows a diagram relating to a non-limiting example of the workflow of the graphic interface according to the invention.

### DETAILED DESCRIPTION

The system for producing a cranial operculum for a living being according to the invention comprises an acquisition device configured to define an area of planned removal of cranial bone (i.e. in a simulation carried out in the pre-surgery phase) on the skull of a living being, a first three-dimensional (or 3D) detection device configured for 3D detection of the skull cap shape and, optionally, of the thickness of the skull to be removed which is included in said area of planned removal of cranial bone, a second detection device configured to detect the points of the margin of the crater in the cranial bone after removal of the part of skull cap included in said area of planned removal, a processing unit configured to process a 3D model of the operculum and send it to an electronic digital fabrication device configured to produce and/or process three-dimensional objects.

In the present description, the term "digital fabrication" (or "fabbing") refers to the process whereby it is possible to create solid three-dimensional objects from digital files. This process exploits different fabrication techniques, both additive (such as 3D printing) and subtractive, such as laser cutting and milling.

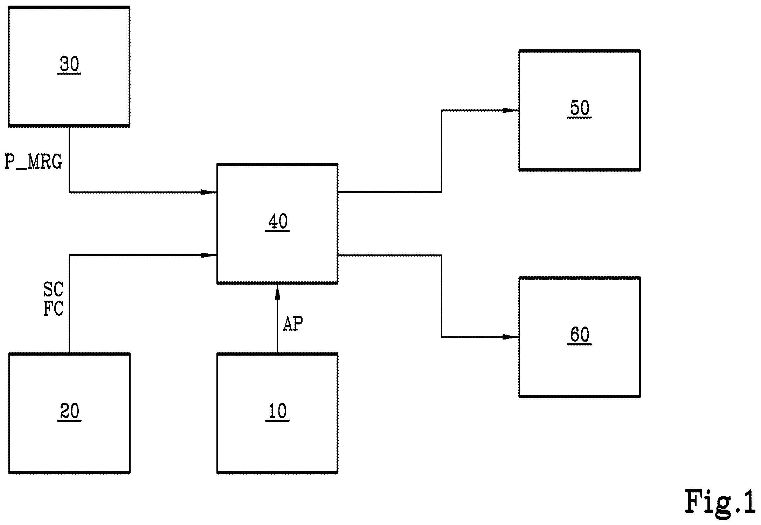

With reference to figure 1, the processing unit 40 is in data communication with the acquisition device 10, with the first three-dimensional detection device 20, with the second detection device 30 for detecting the points P_MRG of the margin 3 of the bone crater 4 and with the electronic digital fabrication device 50.

The processing unit 40 can preferably be connected to a display 60 configured to represent three-dimensional images of the skull 6 of the patient, the edge of the bone crater 4 and/or the cranial operculum 1.

The acquisition device 10 enables the system to approximately determine an area of intervention or planned removal of cranial bone Ap, before the surgical procedure (i.e. pre-surgery simulation phase), based on diagnostic images (for example, computed tomography, nuclear magnetic resonance and variants thereof) of the patient and based on the target zone for the surgical procedure (for example, removal of a primitive or secondary brain tumour, removal of skull base tumours, location of specific areas of the base nuclei for the treatment of functional pathologies).

The system is preferably configured to segment the region of interest (e.g. tumour, oedemas, neoplasms, infections, vascular lesions) and the skull cap. An algorithm will suggest the region of planned removal of the skull cap Ap, which can in any case always be corrected by the physician based on specific needs.

Alternatively, it will be the neurosurgeon who can enter the area of planned removal of cranial bone Ap into the system via a graphic interface, as illustrated in continuation, by tracing, on the segmented 3D reconstruction of the skull cap, the region of planned removal of the skull Ap. Optionally, the neurosurgeon can modify the area of planned removal Ap proposed by the system via the graphic interface. The data related to the area of planned removal of cranial bone Ap from the skull cap are sent to the processing unit 40 by said acquisition device 10.

The first 3D detection device 20 is configured to detect the skull cap shape Fc from one or more diagnostic images of the patient. Optionally, the first device 20 is also capable of detecting the thickness Sc of the portion of skull to be removed from said diagnostic images. By way of non-limiting example, the first device 20 is configured to perform a segmentation of the skull cap, preferably threshold based and adaptive level-set or fast marching.

The data related to the thickness Sc and shape Fc of the skull cap are sent to the processing unit 40 by said first 3D detection device 20. With the simulated data of the region of planned removal of cranial bone Ap, the thickness Sc and the shape Fc of the skull cap, the processing unit 40 is capable of processing a shape estimated in the pre-surgery phase from the skull cap dimensions.

Following removal of the part of skull cap included in said area of planned removal Ap, the second 3D detection device 30 detects the set of points of the margin 3 of the crater in the cranial bone 4, indicated in figure 1 with P_MRG, and sends them to the processing unit 40.

The second detection device 30 for detecting the points of the margin 3 of the crater in the cranial bone 4 preferably comprises an anthropomorphic measuring arm 31 equipped with a sterile tip 32 capable of moving freely and with extreme simplicity and precision on the margin 3 of the area of removed bone 4, in such a way as to detect a set of points P_MRG. The tip 32 can preferably be made of polymer or another sterile, rigid material, such as, for example, steel, ceramics, titanium or aluminium. The anthropomorphic arm can preferably be of the type with 5 or more axes. Every time that the surgeon activates the controls provided, the measuring arm 31 sends to the processing unit 40 the three-dimensional coordinates of the point in space in which the tip of the stylus 32 is located.

As an alternative to the probing arm 31, the set of points P_MRG of the margin 3 of the crater in the cranial bone 4 can be detected by a three-dimensional scanning laser (or scanner) having at least 3 reference axes.

The first detection device 20 is configured to acquire the skull cap shape Fc and, optionally, the skull cap thickness Sc from one or more diagnostic images of the patient.

The first detection device 20 is further configured so as to obtain, from the computed tomography (CT) or from other diagnostic images of the patient (for example MR), first image data, subsequently performing the segmentation.

The first detection device 20 is preferably configured to acquire the skull cap shape Fc by means of a three-dimensional scanning laser (or scanner) having at least 3 reference axes.

The digital fabrication device 50 enables the three-dimensional operculum to be produced by additive fabrication or stereolithography or using subtractive technology, starting from a 3D digital model thereof.

As an alternative to 3D additive printing, the cranial operculum can be produced using subtractive technologies, for example using numerical control (CNC) machines, such as, for example, lathes or milling machines, preferably activatable by the surgeon or specialised hospital staff.

In such a case, based on the shape estimated in the pre-surgery phase from the skull cap dimensions, the processing unit 40 processes and suggests the type of "block of material" of dimensions sufficient to allow the numerical control machine to obtain an operculum of the predefined shape, dimensions and margin by removal of material. The block of material can be supplied inside sterilised bags and contain an RFID identifier containing information related to the production lot and the patient who will be selected to receive the implant.

The processing unit 40 subsequently communicates to the numerical control machine the type of material, dimensions, finish and features it is intended to give to the final piece. During machining, the CNC machine starts from the definitive 3D operculum model file and works on the block of material, which is milled internally or externally with tools differing from one another, depending on the material they are required to machine.

In order to produce the cranial operculum, the additive technology (3D printing) can preferably be combined with the subtractive technology. The combination of the two technologies produces excellent results, since by printing the operculum in 3D (additive technology) it could be envisaged to finish it using subtractive fabrication machines which mill it from the inside (subtractive technology).

The processing unit 40 is configured to process a three-dimensional (3D) digital model of the cranial operculum 1 based on the area of planned removal of cranial bone Ap determined by the acquisition device 10, the skull cap shape Fc and the skull thickness Sc detected by the first 3D detection device 20 and based on the margin 3 of the crater in the cranial bone 4 detected by the second 3D detection device 30.

The thickness Sc of the artificial operculum 1 is preferably uniform and predetermined, sufficient in any case to withstand pressure stresses. In this manner, one achieves the technical effects of rendering the passage of ultrasound for post-surgery imaging more homogeneous and of facilitating the calculations if used as a window for focused ultrasound therapies. Furthermore, the use of a predetermined thickness makes the reconstruction and 3D printing easier, in addition to improving the acoustic properties of the operculum.

In general, it should be noted that in the present context and in the claims herein below, the processing unit 40 is presented as being subdivided into distinct functional modules (memory modules or operating modules) for the sole purpose of describing the functions thereof clearly and thoroughly.

Actually, this processing unit 40 can be constituted by a single electronic device, suitably programmed for performing the functions described, and the various modules can correspond to a hardware entity and/or routine software that are part of the programmed device.

Alternatively or additionally, these functions can be performed by a plurality of electronic devices over which the above-mentioned functional modules can be distributed.

The processing unit 40 can also make use of one or more processors for executing the instructions contained in the memory modules.

Said functional modules can also be distributed over different local or remote computers, depending on the architecture of the network in which they reside.

The systems further comprise all the memory and/or operating means and/or modules necessary for implementing the functions described in the respective methods disclosed.

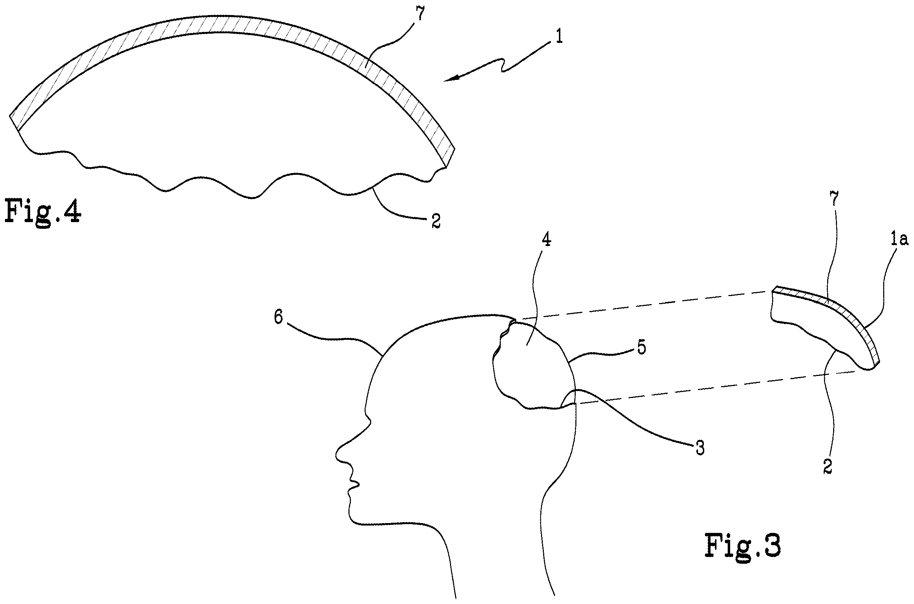

Figure 3 schematically illustrates a lateral view of a patient following a neurosurgical procedure in which a cranial operculum 1a has been removed from the skull 6. In the patient's head one can see the bone defect or craniectomy opening 4, left open following the removal of the cranial operculum 1a, the cranial edge or margin 3 and part of the brain 5. The removed cranial operculum has a thickness 7 and an edge or margin 2. The pattern of the margins 2 and 3, respectively, of the craniectomy opening 4 and of the removed operculum 1a, can prove to be very irregular, due to the method of performing the craniectomy. The craniotomy is planned with the navigation system. Once the margins of the craniotomy have been defined, it can be performed in two ways: a) a drill hole (key-hole) is fashioned, and then with a dissector the dura mater is detached from the inner compact bone (tabula interna ossis cranii), then a craniotome (drill with a lateral sharp tip) is inserted into the hole and the bone is sectioned, or b) the outer compact bone is sectioned with a high-speed drill with a slender tip and then, using a mallet and scalpel, the cortical part and inner compact part of the bone are progressively fractured. The irregularity of the margin 3 of the craniectomy opening makes it difficult to produce an artificial operculum capable of matching it perfectly.

According to the invention, the processing unit 40 comprises a first processing module 410 configured to generate a first three-dimensional digital model M1 of the artificial cranial operculum 1, processed on the basis of the data related to said area of planned removal Ap, said skull thickness Sc and said skull cap shape Fc in said area of planned removal Ap, a second processing module 411 configured to generate a three-dimensional digital margin model M_Mrg on the basis of the points P_Mrg of the margin 3 of the crater in the cranial bone 4 detected by said second detection device 30, a third processing module 412 configured to generate a second three-dimensional digital model M2 of the operculum 1 processed on the basis of said first three-dimensional digital model M1 and said craniectomy margin model M_Mrg and a data transmission module 413 configured to transmit at least said second three-dimensional digital model M2 to said electronic digital fabrication device 50.

Optionally, between said third processing module 412 and said data transmission module 413, it is possible to interpose a memory module 414 configured to memorise at least the second digital model M2 before transmission to the digital fabrication device 50 or to other electronic devices external to the processing unit 40.

The first digital model M1, processed before the surgical procedure, can be used by the neurosurgeon to simulate the removal of the cranial operculum 1a from the skull 6 of the patient and/or to propose to the neurosurgeon some geometries of the artificial operculum 1 that enable different points and fixation methods, leaving it up to the surgeon to choose best type of procedure.

The second processing module 411 is configured to generate a digital margin model 3D M_Mrg on the basis of the set of points of the margin P_Mrg of the crater in the cranial bone 3 acquired by said tip 32 or by said laser scanner.

Optionally, the processing unit 40 comprises a user interface module 414 in data communication with the modules 410, 411, 412 and 416; the user interface module 414 is configured to enable the neurosurgeon to perform one or more of the following operations:
a) to enter or modify the margins of the area of planned removal of cranial bone Ap;
b) to enter or modify the thickness Sc input to the first module 410 or the thickness Sc of at least one between the first M1 and the second M2 three-dimensional digital models;
c) to modify said skull cap shape Fc;
d) to add, remove or move one or more supplementary fixation fins 100, 101;
e) to remove or move a "projecting housing usable as a placeholder for markers";
f) to set the production of said artificial operculum 1 with a non-ultrasound-transparent, radio-opaque texture for CT and/or MR scans;
g) to set the digital fabrication device to be used to produce the cranial operculum. In particular, by means of:
   g1) an additive technology (3D printing);
   g2) alternatively, a subtractive technology (numerical control machine);
   g3) alternatively, the operculum is made by means of 3D printing and subsequently finished with a numerical control machine.

If the subtractive digital fabrication technology is involved, the user interface module will display the type and dimensions of the block of material to be machined in the CNC machine before the surgery phase.

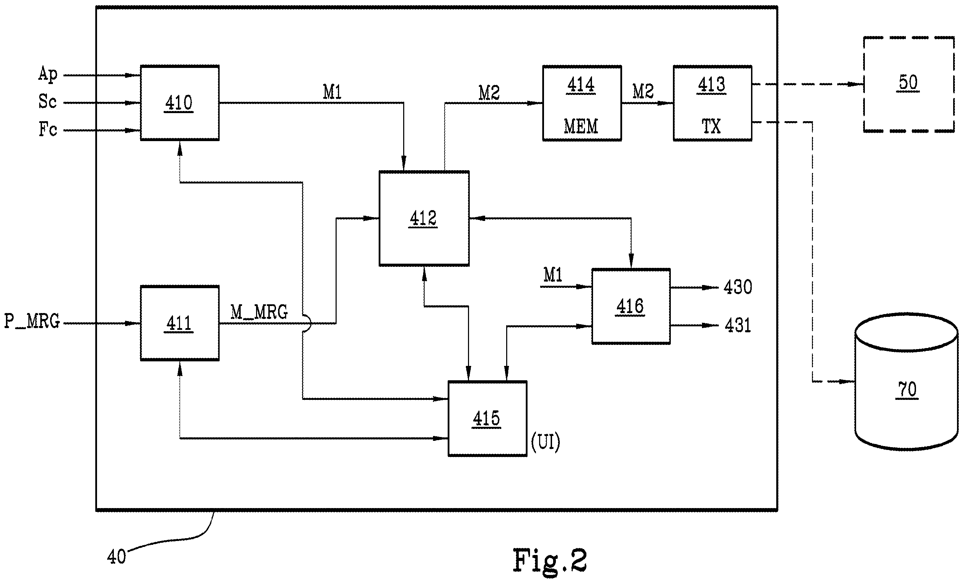

The system can optionally comprise a simulation module 416 configured to simulate the removal of the cranial bone 1a and to define said area of planned removal of cranial bone Ap on the basis of the type of procedure entered by the neurosurgeon. The simulation module 416 can preferably be comprised within the processing unit 40 as illustrated by way of example in figure 2. The simulation module 416 is preferably configured to propose to the surgeon some geometries of the cranial operculum 1 that enable various fixation points, optionally leaving it up to the surgeon to choose the best geometry.

Once the virtual reconstruction of the artificial operculum 1 has been completed, the simulation module 416 preferably allows it to be verified that the 3D model of the missing bone flap (reduced in size if necessary and appropriately smoothed on the surface) fits the 3D model of the skull by means of a graphic assembly of both 3D models. In this manner it is possible to check the correct matching of the artificial operculum 1 with the rest of the skull, as well as its aesthetic impact.

Finally, the system according to the invention can comprise a memory or server 70 in which one or more among said first model M1, said second model M2 and said parameters Ap, Sc, Fc associated with the data of the living being are memorised. By way of example, the data memorisable in the memory 70 also comprise: personal data, medical history and diagnosis of the patient, a patient file according to the standard "Digital Imaging and Communications in Medicine" (or DICOM format), reconstructed 3D volume of the diagnostic images, 3D volume of the artificial operculum generated and segmentation of the skull.

The processing unit 40 is preferably connected to the 3D printer or milling device 50, the server 70 and other electronic devices via a computer network.

The artificial cranial operculum 1 produced by the 3D printer or milling machine 50 consists of a cap made of rigid material that will replace a bone operculum 1a removed following a craniotomy, or following the procedure that enables access to the inside of the skull 6 of a living being in order to remove a brain tumour or reconstruct damaged parts of the skull.

For this reason, the artificial cranial operculum 1 must be an exact replica of the native bone operculum 1a so as to be able to fit perfectly with the rest of the skull 6. Preferably, the thickness of the artificial operculum 1 can be uniform and predetermined, without having to follow the thickness of the native bone. In order for the artificial operculum to be metabolised without any harmful effect for the patient, it is necessary for it to be made of a biocompatible material and it must likewise be sterilisable prior to application.

The artificial operculum 1 according to the invention can be produced by the electronic digital fabrication device 50 in such a way as to be able to incorporate and interface with one or more therapeutic and diagnostic instruments, i.e. "modules", while at the same time maintaining the ability to monitor the patient's brain using an ultrasound instrument. In particular, use can be made of existing intracranial monitoring devices or ones specifically developed for use with the artificial operculum.

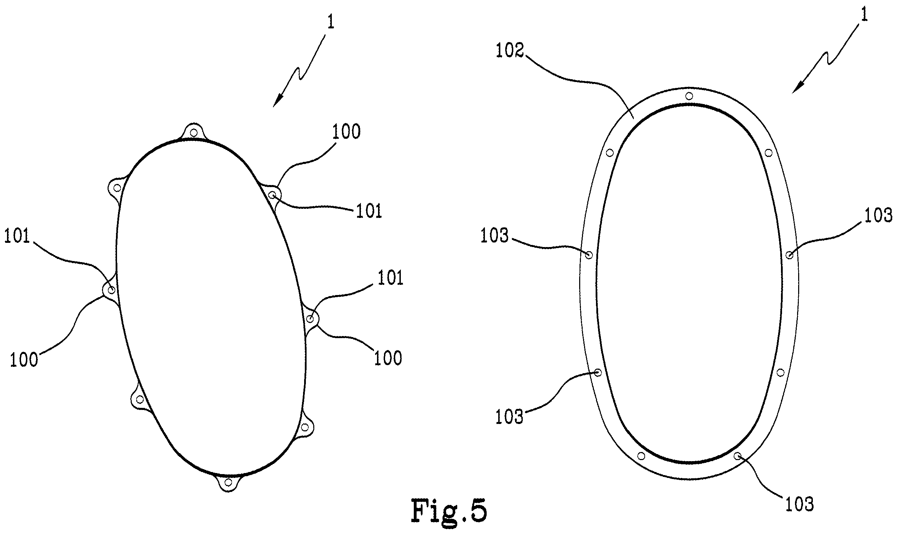

As shown in figure 5, the artificial operculum 1 can be produced by the electronic digital fabrication device 50 with the presence of perimeter fins 100, 102 having holes 101, 103 suitable for securing it to the skull 6 by means of suture thread. In particular, depending on the cranial zone of intervention, the artificial operculum 1 can be produced with at least two or more fins 100, each endowed with one or more holes 101, or else with a single perimeter fin 102 having at least two through holes 103.

Optionally, the artificial operculum 1 can be produced by the electronic digital fabrication device 50 with the presence of a texture visualisable by means of computed tomography (CT) and magnetic resonance (MR) in order to provide a specific and precise reference of the anatomical structures inside the artificial operculum. The texture must not be dense, but such as to enable the use of ultrasound.

With the system according to the invention, it is likewise possible to produce the artificial operculum 1 with the presence of known attachment points ("placeholders") which serve to position a marker for augmented reality, also, but not only, in reference to the visible texture as per the previous paragraph. Examples of markers that can be housed in the placeholder are crosses, Aruco markers and the like.

Optionally, the artificial operculum 1 can be produced in such a way as to incorporate at least one pressure and temperature sensor activatable by induction also through portable instruments. In such a manner, it is possible to enable monitoring of the endocranial pressure and temperature.

As an alternative to the above-mentioned pressure sensor, it is possible to incorporate a pressure sensor characterised by a fin made of polymethylmethacrylate (abbreviated as PMMA) or another non-ultrasound-transparent material, whose position varies with variations in the intracranial pressure. This sensor renders the pressure variation visualisable by means of a simple ultrasound scan, without the need to use an electronic apparatus or complex devices.

Once the artificial operculum 1 has been produced, before the surgical use thereof, it is automatically transferred into a sterilisation chamber that is an integral part of the body of the digital fabrication machine 50. Through sterilisation methods (UV or gamma radiation or gas plasma), the artificial opercula produced, already intrinsically sterile given the fabrication process and materials used, are further sterilised to eliminate the presence of any contaminants that may have contacted the printing surface. Optionally, the artificial opercula may be produced in a number of copies, and each operculum is automatically transferred into a sterile bag and sealed.

In the follow-up phase the surgeon can use an ultrasound scan, optionally fusing, thanks to the various modules of the processing unit 40, the ultrasound images with CT or MR images. This fusion is facilitated and referenced thanks to the presence of the "texture" as per the previous passages.

In the event of the need for intervention, the system is capable of converting the diagnostic image into a 3D volume from which it is then possible to acquire the skull cap shape Fc and, optionally, the skull thickness Sc to be removed.

Optionally, the system can activate an augmented reality function through the use of appropriate markers that can be housed in the above-mentioned placeholders. The surgeon can thus focus on the marker and, via the system functions, visualise, also for training purposes, the corresponding three-dimensional images overlaid on the patient's skull.

The artificial cranial operculum is produced by using polymeric materials such as, for example, polyethylene (PE), polystyrene or polymethylpentene (TPX), or polyethylene terephthalate (PET). Preferably, a high-density polyethylene (HDPE 300 - High Density PE) can be used.

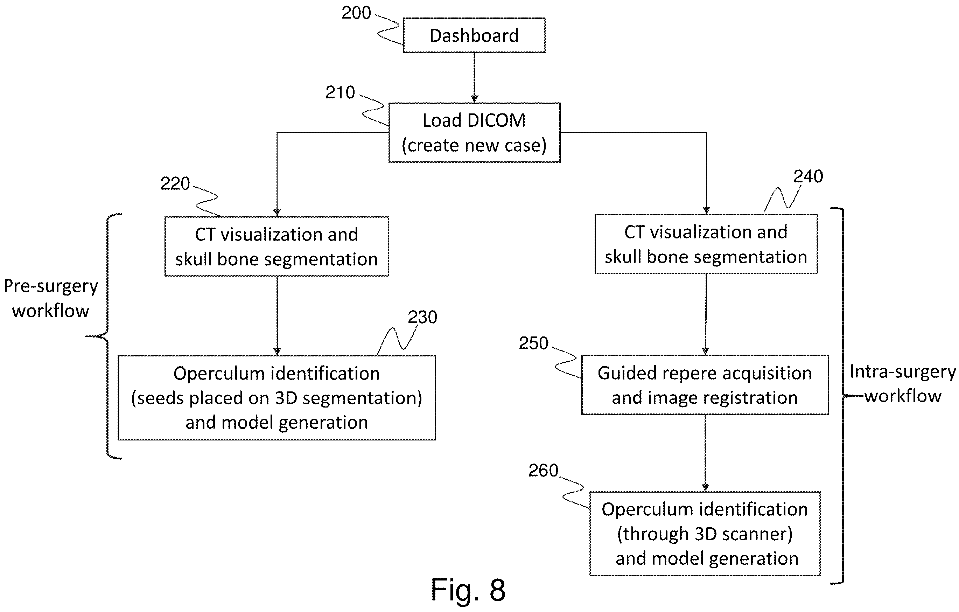

Figure 8 illustrates a non-limiting example of a graphic interface of the system (or workflow of the various steps) for producing a cranial operculum 1 for a living being according to the present invention.

The block or step 200 presents the physician (or another health worker) with a list of patients, along with the corresponding DICOM files and their creation date. The user can manage the list, for example by deleting or adding new patients. Furthermore, by selecting a given patient from the list, it will be possible to go to the simulation branch (pre-surgery phase) or to the branch of the sequence of steps carried out in the surgery phase, which eventually lead to the production of the cranial operculum during the procedure. In the block or step 210 it is possible to load the DICOM file of a given patient. An external file can be loaded or the file can be supplied from one of the above-described devices.

In the pre-surgery simulation branch, in the block or step 220 the computed tomography image is visualised and the cranial bone of the patient is segmented. In the block 230 the anatomical or artificial operculum landmarks (points of repere) are identified and the operculum model is generated (in the pre-surgery phase), with the associated dimensions and characteristics.

In the "surgery" branch of figure 8, the margin and size and shape of the cranial crater are acquired following removal in the operating room, along with a 3D scan (for example, by means of a 3D scanner) of the cranial operculum extracted from the patient, so as to have real data acquired in the operating room.

The system will call up and display the model of the operculum simulated in the pre-surgery phase and adapt it (image registration) based on the data acquired in situ during the procedure in the operating room.

The system for producing a cranial operculum 1 for a living being comprises an acquisition device 10 configured to define an area of planned removal of cranial bone Ap on the skull 6 of the living being and a first 3D detection device 20 configured for 3D detection of the shape Fc of the skull cap to be removed which is included in said area of planned removal of cranial bone Ap. The devices 10 and 20 are related to a pre-surgery simulation phase that takes place before the actual removal of the cranial operculum of the patient. The system further comprises a second detection device 30 configured to detect the points P_MRG of the margin of the crater in the cranial bone 3 following removal of the part of skull cap included in said area of planned removal Ap; an electronic digital fabrication device (i.e. via 3D printing or milling, "digital fabrication") 50 configured to produce three-dimensional objects; a processing unit 40 in data communication with said acquisition device 10, said first 3D detection device 20, said second detection device 30 for detecting the points of the margin 2 of the crater in the cranial bone 3 and said electronic digital fabrication device 50, wherein said processing unit 40 is configured to define (prior to a surgical procedure of craniotomy) a 3D digital model of the cranial operculum 1 based on the skull cap shape Fc, skull thickness Sc detected by said first 3D detection device 20 and based on the margin 2 of the crater in the cranial bone 3 detected by said second detection device 3D 30, the processing unit 40 comprising: a first processing module 410 configured to generate (prior to the performance of a surgical procedure of craniotomy) a first 3D digital model M1 of the cranial operculum 1 processed on the basis of said area of planned removal Ap, said skull thickness Sc and said skull cap shape Fc in said area of planned removal Ap; a second processing module 411 configured to generate (in the intra-surgery phase, following removal of part of the skull cap) a 3D digital margin model M_Mrg on the basis of the points P_MRG of the margin 2 of the crater in the cranial bone 3 detected by said second detection device 30; a third processing module 412 configured to generate a second digital model 3D M2 of the operculum 1 processed on the basis of said first digital model 3D M1 and said craniectomy margin model M_Mrg; and a transmission module 413 configured to transmit said second digital model 3D M2 to said electronic digital fabrication device 50.

Also discloses herein although not forming part of the claimed invention is a method for producing a cranial operculum 1 for a living being comprising the steps of: before carrying out a surgical procedure of craniotomy,
- defining an area of planned removal of cranial bone Ap on the skull 6 of the living being;
- detecting the skull thickness Sc in said area of planned removal of cranial bone Ap;
- detecting the 3D shape Fc of the skull cap to be removed in said area of planned removal of cranial bone Ap;
- generating a first 3D digital model M1 of the cranial operculum 1 processed on the basis of said area of planned removal Ap, said skull thickness Sc and said skull cap shape Fc in said area of planned removal Ap;
after removing a part of the skull cap 1a in the area of planned removal of cranial bone Ap,
- detecting the points of the margin 2 of the crater in the cranial bone 3;
- generating a 3D digital margin model M_Mrg of the removed skull area;
- generating a second 3D digital model M2 of the cranial operculum 1 processed on the basis of said first 3D digital model M1 and said digital margin model 3D M_Mrg;
- sending said second digital model 3D M2 to a digital electronic fabrication device (i.e. 3D printing or milling) 50; and
- making at least one copy of said cranial operculum 1 by means of said electronic digital fabrication device 50.

The method for producing a cranial operculum 1 optionally comprises a simulation step for simulating the removal of cranial bone 1a and defining said area of planned removal of cranial bone Ap on the basis of the type of procedure entered by the neurosurgeon before the surgical procedure of craniectomy.

The step of detecting the skull thickness Sc in said area of planned removal of cranial bone Ap can be carried out by using a probing arm 31 equipped with a tip 32 which is made to pass over the margin 2 of the area of removed bone.

Alternatively, the steps of detecting the skull cap shape Fc or the set of points P_Mrg of the margin 2 of the crater in the cranial bone 3 are carried out by means of a three-dimensional scanning laser (or scanner) having at least 3 reference axes.

In a further alternative, the steps of detecting the skull cap shape Fc or the skull thickness Sc are achieved by means of one or more diagnostic images of the living being.

## Claims

1. A system for producing a cranial operculum (1) for a living being, comprising:
an acquisition device (10) configured to define an area of planned removal of cranial bone (Ap) on the skull (6) of the living being based on diagnostic images of the patient and on the target zone for a surgical procedure;
a first 3D detection device (20) configured for 3D detection of a skull cap shape (Fc) of a skull cap to be removed within said area of planned removal of cranial bone (Ap);
a second detection device (30) configured to detect margin points (P_MRG) of a margin of a crater in the cranial bone (3) following removal of a part of skull cap included in said area of planned removal (Ap);
an electronic digital fabrication device (50) configured to produce three-dimensional objects; and
a processing unit (40) in data communication with said acquisition device (10), said first 3D detection device (20), said second detection device (30) for detecting the points of the margin (2) of the crater in the cranial bone (3) and said 3D electronic digital fabrication device (50),
wherein said processing unit (40) is configured to define a 3D digital model of the cranial operculum (1) based on the skull cap shape (Fc) and the skull thickness (Sc) detected by said first 3D detection device (20) and based on the margin (2) of the crater in the cranial bone (3) detected by said second 3D detection device (30), the processing unit (40) comprising:
• a first processing module (410) configured to generate a first 3D digital model (M1) of the cranial operculum (1) processed on the basis of said area of planned removal (Ap), said skull thickness (Sc) and said skull cap shape (Fc) in said area of planned removal (Ap) wherein said first 3D digital model (M1) of the cranial operculum (1) is generated before carrying out a surgical procedure of craniotomy;
• a second processing module (411) configured to generate a 3D digital margin model (M_Mrg) on the basis of the points (P_MRG) of the margin (2) of the crater in the cranial bone (3) detected by said second detection device (30);
• a third processing module (412) configured to generate a second 3D digital model (M2) of the operculum (1) processed on the basis of said first 3D digital model (M1) and said craniectomy margin model (M_Mrg); and
• a transmission module (413) configured to transmit said second 3D digital model (M2) to said electronic digital fabrication device (50).

2. The system according to claim 1, wherein said processing unit (40) comprises a simulation module (416) configured to simulate the removal of the cranial bone (3) and to define said area of planned removal of the cranial bone (Ap) on the basis of the type of procedure entered by the neurosurgeon.

3. The system according to claim 1 or 2, wherein said second detection device (30) for detecting the points (P_MRG) of the margin (2) of the crater in the cranial bone (3) comprises a probing arm (31) equipped with a tip (32) which is made to pass over the margin (2) of the area of removed bone.

4. The system according to claim 3, wherein said second processing module (411) is configured to generate a 3D digital margin model (M_MRG) on the basis of the set of points of the margin (2) of the crater in the cranial bone (3) acquired by said tip (32).

5. The system according to one or more of the preceding claims, wherein said skull cap shape (Fc) or said set of points of the margin (2) of the crater in the cranial bone (3) is detected by a three-dimensional scanning laser (or scanner) having at least 3 reference axes.

6. The system according to one or more of the preceding claims, wherein said skull cap shape (Fc) or said skull cap thickness (Sc) is detected via one or more diagnostic images.

7. The system according to one or more of the preceding claims, wherein said skull cap thickness (Sc) is uniform and predetermined.

8. The system according to one or more of the preceding claims, wherein said processing unit (40) comprises a user interface (414) configured to enable the neurosurgeon to perform one or more of the following operations:
a) to enter or modify the margins of the area of planned removal of the cranial bone (Ap);
b) to enter or modify the thickness (Sc) input to said first module (410) or the thickness (Sc) of at least one between said first (M1) and said second (M2) 3D digital model;
c) to modify said skull cap shape (Fc);
d) to add, remove or move one or more supplementary fixation fins (100, 103) from at least one between said first (M1) and said second (M2) 3D digital model;
e) to remove or move one or more projecting housings usable as placeholders for markers;
f) to set the 3D printing of said operculum (1) with a non-ultrasound-transparent, radio-opaque texture for computed tomography (CT) and/or magnetic resonance (MR) scans.

9. The system according to one or more of the preceding claims, wherein said digital fabrication device is adapted to produce said cranial operculum (1) with one or more of the following characteristics:
a) by means of the use of ultrasound-transparent materials;
b) by means of a non-ultrasound-transparent, radio-opaque texture for computed tomography (CT) and/or magnetic resonance (MR) scans;
c) by means of one or more supplementary fixation fins (100, 103);
d) by means of one or more projecting housings suitable for housing one or more markers.

10. The system according to one or more of the preceding claims, comprising a memory (70) in which one or more among said first model (M1), said second model (M2) and said parameters (Ap, Sc, Fc) associated with the data of the living being are memorised.

## Patentansprüche

1. System zur Herstellung eines Operculums (1) für ein Lebewesen, umfassend:
eine Erfassungsvorrichtung (10), die ausgelegt ist, um einen Bereich einer geplanten Entfernung eines Schädelknochens (Ap) auf dem Schädel (6) des Lebewesens basierend auf diagnostischen Bildern des Patienten und auf der Zielzone für ein chirurgisches Verfahren zu definieren;
eine erste 3-D-Erkennungsvorrichtung (20), die für die 3-D-Erkennung einer Schädeldachform (Fc) eines zu entfernenden Schädeldachs innerhalb des Bereichs der geplanten Entfernung eines Schädelknochens (Ap) ausgelegt ist;
eine zweite Erkennungsvorrichtung (30), die ausgelegt ist, um Randpunkte (P_MRG) eines Rands eines Kraters im Schädelknochen (3) nach der Entfernung eines Schädeldachteils, das im Bereich der geplanten Entfernung (Ap) eingeschlossen ist, zu erkennen;
eine elektronische, digitale Herstellungsvorrichtung (50), die ausgelegt ist, um dreidimensionale Objekte zu produzieren, und
eine Verarbeitungseinheit (40) in Datenkommunikation mit der Erfassungsvorrichtung (10), der ersten 3-D-Erkennungsvorrichtung (20), der zweiten Erkennungsvorrichtung (30) zum Erkennen der Punkte des Rands (2) des Kraters im Schädelknochen (3) und der elektronischen, digitalen 3-D-Herstellungsvorrichtung (50),
wobei die Verarbeitungseinheit (40) ausgelegt ist, um ein digitales 3-D-Modell des Operculums (1) basierend auf der Schädeldachform (Fc) und der Schädeldicke (Sc), erkannt von der ersten 3-D-Erkennungsvorrichtung (20), und basierend auf dem Rand (2) des Kraters im Schädelknochen (3), erkannt von der zweiten 3-D-Erkennungsvorrichtung (30), zu definieren, wobei die Verarbeitungseinheit (40) Folgendes umfasst:
• ein erstes Verarbeitungsmodul (410), das ausgelegt ist, um ein erstes digitales 3-D-Modell (M1) des Operculums (1) zu generieren, verarbeitet auf Basis des Bereichs der geplanten Entfernung (Ap), der Schädeldicke (Sc) und der Schädeldachform (Fc) im Bereich der geplanten Entfernung (Ap), wobei das erste digitale 3-D-Modell (M1) des Operculums (1) generiert wird, bevor ein chirurgisches Kraniotomieverfahren durchgeführt wird;
• ein zweites Verarbeitungsmodul (411), das ausgelegt ist, um ein digitales 3-D-Randmodell (M_Mrg) auf Basis der Punkte (P_MRG) des Rands (2) des Kraters im Schädelknochen (3), erkannt von der zweiten Erkennungsvorrichtung (30), zu generieren;
• ein drittes Verarbeitungsmodul (412), das ausgelegt ist, um ein zweites digitales 3-D-Modell (M2) des Operculums (1) zu generieren, verarbeitet auf Basis des ersten digitalen 3-D-Modells (M1) und des Kraniotomierandmodells (M_Mrg), und
• ein Übertragungsmodul (413), das ausgelegt ist, um das zweite digitale 3-D-Modell (M2) an die elektronische, digitale Herstellungsvorrichtung (50) zu übertragen.

2. System nach Anspruch 1, wobei die Verarbeitungseinheit (40) ein Simulationsmodul (416) umfasst, das ausgelegt ist, um die Entfernung des Schädelknochens (3) zu simulieren und den Bereich der geplanten Entfernung des Schädelknochens (Ap) auf Basis des vom Neurochirurgen gewählten Verfahrenstyps zu simulieren.

3. System nach Anspruch 1 oder 2, wobei die zweite Erkennungsvorrichtung (30) zum Erkennen der Punkte (P_MRG) des Rands (2) des Kraters im Schädelknochen (3) einen Fühlerarm (31) umfasst, der mit einer Spitze (32) ausgestattet ist, die über den Rand (2) des Bereichs des entfernten Knochens geführt wird.

4. System nach Anspruch 3, wobei das zweite Verarbeitungsmodul (411) ausgelegt ist, um ein digitales 3-D-Randmodell (M_MRG) auf Basis der Reihe von Punkten des Rands (2) des Kraters im Schädelknochen (3), die von der Spitze (32) erfasst wurden, zu generieren.

5. System nach einem oder mehreren der vorhergehenden Ansprüche, wobei die Schädeldachform (Fc) oder die Reihe von Punkten des Rands (2) des Kraters im Schädelknochen (3) von einem dreidimensionalen Scan-Laser (oder Scanner), aufweisend mindestens 3 Referenzachsen, erkannt wird.

6. System nach einem oder mehreren der vorhergehenden Ansprüche, wobei die Schädeldachform (Fc) oder die Schädeldachdicke (Sc) mittels eines oder mehrerer diagnostischer Bilder erkannt wird.

7. System nach einem oder mehreren der vorhergehenden Ansprüche, wobei die Schädeldachdicke (Sc) einheitlich und vorgegeben ist.

8. System nach einem oder mehreren der vorhergehenden Ansprüche, wobei die Verarbeitungseinheit (40) eine Benutzerschnittstelle (414) umfasst, die ausgelegt ist, um dem Neurochirurgen zu ermöglichen, einen oder mehrere der folgenden Vorgänge durchzuführen:
a) Eingabe oder Änderung der Ränder des Bereichs der geplanten Entfernung des Schädelknochens (Ap);
b) Eingabe oder Änderung der in das erste Modul (410) eingegebenen Dicke (Sc) oder der Dicke (Sc) von mindestens entweder dem ersten (M1) und/oder dem zweiten (M2) digitalen 3-D-Modell;
c) Änderung der Schädeldachform (Fc);
d) Hinzufügung, Entfernung oder Bewegung von einer oder mehreren zusätzlichen Fixierrippen (100, 103) von mindestens entweder dem ersten (M1) und/oder dem zweiten (M2) digitalen 3-D-Modell;
e) Entfernung oder Bewegung von einem oder mehreren hervorspringenden Aufnahmen, die als Platzhalter für Marker nutzbar sind;
f) Einstellung des 3-D-Drucks des Operculums (1) mit einer nicht ultraschalldurchlässigen, strahlenundurchlässigen Textur für Scans der Computertomographie (CT) und/oder Magnetresonanz (MR).

9. System nach einem oder mehreren der vorhergehenden Ansprüche, wobei die digitale Herstellungsvorrichtung eingerichtet ist, um das Operculum (1) mit einem oder mehreren der folgenden Merkmale zu produzieren:
a) mittels der Nutzung von ultraschalldurchlässigen Materialien;
b) mittels einer nicht ultraschalldurchlässigen, strahlenundurchlässigen Textur für Scans der Computertomographie (CT) und/oder Magnetresonanz (MR);
c) mittels einer oder mehrerer zusätzliche Fixationsrippen (100, 103);
d) mittels einer oder mehrerer hervorspringender Aufnahmen, die geeignet sind, um einen oder mehrere Marker aufzunehmen.

10. System nach einem oder mehreren der vorhergehenden Ansprüche, umfassend einen Speicher (70), in dem entweder das erste Modell (M1) und/oder das zweite Modell (M2) und/oder die Parameter (Ap, Sc, Fc), die mit den Daten des Lebewesens assoziiert sind, gespeichert sind.

## Revendications

1. Système de réalisation d'un opercule crânien (1) pour un être vivant, comprenant :
un dispositif d'acquisition (10) configuré pour définir une zone d'ablation planifiée d'os crânien (Ap) sur le crâne (6) de l'être vivant sur la base d'images diagnostiques du patient et de la zone cible d'une intervention chirurgicale ;
un premier dispositif de détection 3D (20) configuré pour la détection 3D d'une forme de boîte crânienne (Fc) d'une boîte crânienne à retirer à l'intérieur de ladite zone d'ablation planifiée d'os crânien (Ap) ;
un deuxième dispositif de détection (30) configuré pour détecter des points de marge (P_MRG) d'une marge d'un cratère dans l'os crânien (3) suite à l'ablation d'une partie de la boîte crânienne incluse dans ladite zone d'ablation planifiée (Ap) ;
un dispositif de fabrication électronique numérique (50) configuré pour produire des objets tridimensionnels ; et
une unité de traitement (40) en communication de données avec ledit dispositif d'acquisition (10), ledit premier dispositif de détection 3D (20), ledit deuxième dispositif de détection (30) pour détecter les points de marge (2) du cratère dans l'os crânien (3) et ledit dispositif de fabrication 3D numérique électronique (50),
dans lequel ladite unité de traitement (40) est configurée pour définir un modèle numérique 3D de l'opercule crânien (1) sur la base de la forme de la boîte crânienne (Fc) et de l'épaisseur du crâne (Sc) détectées par ledit premier dispositif de détection 3D (20) et sur la base de la marge (2) du cratère dans l'os crânien (3) détectée par ledit deuxième dispositif de détection 3D (30), l'unité de traitement (40) comprenant :
• un premier module de traitement (410) configuré pour générer un premier modèle numérique 3D (M1) de l'opercule crânien (1) traité sur la base de ladite zone d'ablation planifiée (Ap), de ladite épaisseur du crâne (Sc) et de ladite forme de la boîte crânienne (Fc) dans ladite zone d'ablation planifiée (Ap), dans lequel ledit premier modèle numérique 3D (M1) de l'opercule crânien (1) est généré avant que ne soit effectuée une intervention chirurgicale de craniotomie ;
• un deuxième module de traitement (411) configuré pour générer un modèle de marge numérique 3D (M_Mrg) sur la base des points (P_MRG) de la marge (2) du cratère dans l'os crânien (3) détecté par ledit deuxième dispositif de détection (30) ;
• un troisième module de traitement (412) configuré pour générer un deuxième modèle numérique 3D (M2) de l'opercule (1) traité sur la base dudit premier modèle numérique 3D (M1) et dudit modèle de marge de craniectomie (M_Mrg) ; et
• un module de transmission (413) configuré pour transmettre ledit deuxième modèle numérique 3D (M2) audit dispositif de fabrication électronique numérique (50) .

2. Système selon la revendication 1, dans lequel ladite unité de traitement (40) comprend un module de simulation (416) configuré pour simuler l'ablation de l'os crânien (3) et pour définir ladite zone d'ablation planifiée de l'os crânien (Ap) sur la base du type d'intervention saisi par le neurochirurgien.

3. Système selon la revendication 1 ou 2, dans lequel ledit deuxième dispositif de détection (30) pour détecter les points (P_MRG) de la marge (2) du cratère dans l'os crânien (3) comprend un bras de sondage (31) équipé d'une pointe (32) qui est faite pour passer sur la marge (2) de la zone de l'os retiré.

4. Système selon la revendication 3, dans lequel ledit deuxième module de traitement (411) est configuré pour générer un modèle de marge numérique 3D (M_MRG) sur la base de l'ensemble des points de la marge (2) du cratère dans l'os crânien (3) acquis par ladite pointe (32).

5. Système selon une ou plusieurs des revendications précédentes, dans lequel ladite forme de la boîte crânienne (Fc) ou ledit ensemble de points de la marge (2) du cratère dans l'os crânien (3) est détecté par un laser (ou scanner) à balayage tridimensionnel comportant au moins 3 axes de référence.

6. Système selon une ou plusieurs des revendications précédentes, dans lequel ladite forme de la boîte crânienne (Fc) ou ladite épaisseur de la boîte crânienne (Sc) est détectée par une ou plusieurs images diagnostiques.

7. Système selon une ou plusieurs des revendications précédentes, dans lequel l'épaisseur de la boîte crânienne (Sc) est uniforme et prédéterminée.

8. Système selon une ou plusieurs des revendications précédentes, dans lequel ladite unité de traitement (40) comprend une interface utilisateur (414) configurée pour permettre au neurochirurgien d'effectuer une ou plusieurs des opérations suivantes :
a) saisir ou modifier les marges de la zone d'ablation planifiée de l'os crânien (Ap) ;
b) saisir ou modifier l'épaisseur (Sc) entrée dans ledit premier module (410) ou l'épaisseur (Sc) d'au moins un modèle entre ledit premier (M1) et ledit deuxième (M2) modèle numérique 3D ;
c) modifier ladite forme de la boîte crânienne (Fc) ;
d) ajouter, retirer ou déplacer une ou plusieurs ailettes de fixation (100, 103) supplémentaires à partir d'au moins un modèle entre ledit premier (M1) et ledit deuxième (M2) modèle numérique 3D ;
e) retirer ou déplacer un ou plusieurs logements en saillie utilisables comme paramètres fictifs de marqueurs ;
f) régler l'impression 3D dudit opercule (1) avec une texture non transparente aux ultrasons et radio-opaque pour les examens par tomodensitométrie (CT) et/ou par résonances magnétiques (RM).

9. Système selon une ou plusieurs des revendications précédentes, dans lequel ledit dispositif de fabrication numérique est adapté pour réaliser ledit opercule crânien (1) avec l'une ou plusieurs des caractéristiques suivantes :
a) au moyen de l'utilisation de matériaux transparents aux ultrasons ;
b) au moyen d'une texture non transparente aux ultrasons et radio-opaque pour les examens par tomodensitométrie (CT) et/ou par résonances magnétiques (RM) ;
c) au moyen d'une ou plusieurs ailettes de fixation (100, 103) supplémentaires ;
d) au moyen d'un ou de plusieurs logements en saillie adaptés pour loger un ou plusieurs marqueurs.

10. Système selon une ou plusieurs des revendications précédentes, comprenant une mémoire (70) dans laquelle sont mémorisés un ou plusieurs modèles parmi ledit premier modèle (M1), ledit deuxième modèle (M2) et lesdits paramètres (Ap, Sc, Fc) associés aux données de l'être vivant.
